# FASCICULE DE BREVET EUROPEEN

(11) **EP 3 927 438 B1**
(45) Date de publication et mention de la délivrance du brevet: **19.06.2024**
(21) Numéro de dépôt: 20710234.4
(22) Date de dépôt: 17.02.2020
(51) Int. Cl.: A62B 18/08

(54) **ÉQUIPEMENT RESPIRATOIRE POUR AÉRONEF, ENSEMBLE RESPIRATOIRE ET PROCÉDÉ POUR RANGER L'ÉQUIPEMENT RESPIRATOIRE**
BEATMUNGSAUSRÜSTUNG FÜR EIN FLUGZEUG, BEATMUNGSANORDNUNG UND VERFAHREN ZUM VERSTAUEN DER BEATMUNGSAUSRÜSTUNG
BREATHING EQUIPMENT FOR AN AIRCRAFT, BREATHING ASSEMBLY AND METHOD FOR STOWING THE BREATHING EQUIPMENT

(30) Priorité: 18.02.2019 FR 1901609
(43) Date de publication de la demande: 29.12.2021
(73) Titulaire: SAFRAN AEROTECHNICS, 78370 Plaisir (FR)
(72) Inventeur: SIBUET, Jean-Philippe, 77550 Moissy-Cramayel (FR); BESSON, Alexandre, 77550 Moissy-Cramayel (FR)
(74) Mandataire: Plasseraud IP
(86) Numéro de dépôt international: PCT/FR2020/050289
(87) Numéro de publication internationale: WO 2020/169912

(56) Documents cités:
- US-A- 5 957 132

## Description

### Domaine de l'invention

L'invention concerne un équipement respiratoire pour aéronef, un ensemble respiratoire et un procédé pour ranger un équipement respiratoire dans un aéronef. Plus précisément, ledit ensemble respiratoire est du type comprenant essentiellement un équipement respiratoire muni d'un harnais gonflable et une boite de rangement.

Plus particulièrement, l'équipement respiratoire comprend un masque respiratoire comportant une coque. La coque présente une cavité globale, la coque pouvant notamment comprendre un couvre face oronasal présentant une première cavité et un support supportant une lentille transparente formant un ensemble usuellement dénommée lunette, et présentant une deuxième cavité.

### Etat de la technique

L'équipement respiratoire habituellement utilisé par les membres de l'équipage de l'aéronef doit être mis sûrement et rapidement sur la tête du pilote, à titre préventif ou en situation d'urgence. Cette opération ne doit nécessiter qu'une seule main, car l'autre main est souvent requise pour d'autres tâches essentielles.

Par exemple, en raison d'une panne de pressurisation ou autre problème technique générant une dépressurisation de la cabine de l'aéronef, en raison d'une présence de fumée et/ou de gaz toxique, ou plus généralement en situation d'urgence, le pilote d'un avion doit rapidement mettre son équipement respiratoire destiné à lui fournir l'oxygène nécessaire pour respirer, et il doit souvent le faire d'une main tandis que son autre main est occupée à contrôler l'avion. Ainsi, l'utilisation d'un harnais de tête gonflable a été suggérée pour permettre l'insertion de l'équipement respiratoire d'une seule main. Généralement, le harnais de tête est formé par un ou plusieurs anneaux en tube élastiquement longitudinalement étirables. Le harnais a sensiblement une forme de dôme ou une forme annulaire et est dilaté diamétralement par l'introduction de gaz sous pression pour provoquer l'augmentation de taille du harnais afin qu'il puisse être positionné sur la tête d'un utilisateur. Le flux de gaz est contrôlé par une soupape attachée à l'équipement respiratoire et, après agrandissement du harnais, l'équipement respiratoire est placé contre le visage de l'utilisateur, le harnais étant étendu et espacé de l'arrière de la tête. Une fois que l'équipement respiratoire est correctement positionné, la pression dans le harnais est relâchée, ce qui amène le harnais à se contracter par retour élastique et à venir au contact de la tête du pilote (en l'enserrant légèrement), de sorte que l'équipement respiratoire est bien maintenu dans sa position correcte. Pendant ce temps, l'autre main du pilote est libre de contrôler l'avion ou d'effectuer d'autres tâches qui peuvent être nécessaires.

L'espace pour ranger l'équipement respiratoire étant généralement restreint dans un aéronef, le rangement de l'équipement respiratoire dans une boite de rangement de manière appropriée n'est pas aisé. Or, une position de rangement inappropriée de l'équipement respiratoire risque de ne pas permettre un déploiement satisfaisant du harnais lors de son gonflage.

En particulier, lors du rangement de l'équipement respiratoire dans un dispositif de rangement, le harnais doit se trouver à l'extérieur de la (des) cavité(s). Ainsi, le risque d'avoir un pli du harnais inséré dans la (l'une des) cavité(s) de la coque lorsque le harnais commence à se gonfler est réduit, de sorte que le risque d'un problème de gonflement du harnais est réduit.

Il est déjà connu du document US2018/0361179A1 un système actif, avec une liaison libérable entre le harnais et la boite de rangement. La liaison libérable va forcer l'extraction du harnais, tout en pilotant l'ouverture de l'alimentation en oxygène. Ce système impose une modification non seulement de l'équipement respiratoire, mais également de la boite de rangement.

Il est par ailleurs connu du document US2019/022434A1 un système "semi actif" dans lequel une sangle sur le harnais est utilisée pour le rangement. Un embout spécifique à l'extrémité de cette sangle vient s'interfacer avec les portes de la boite. Si l'embout est bien positionné, les portes peuvent se refermer. Si l'embout n'est pas bien positionné, et donc le harnais mal rangé, les portes ne se referment pas, indiquant que le rangement n'est pas satisfaisant et est à refaire.

US5957132A décrit un équipement selon le préambule de la revendication 1 et un procédé selon le préambule de la revendication 12.

Il est proposé un équipement respiratoire dont l'ergonomie est améliorée pour faciliter un rangement approprié.

### Exposé de la divulgation

L'équipement respiratoire est destiné à fournir un gaz respiratoire à un utilisateur et comporte :
- un masque respiratoire comprenant une coque, la coque présente une cavité et est adaptée pour s'appliquer sur la tête de l'utilisateur autour de la bouche et du nez de l'utilisateur, la coque supportant une lentille transparente et comprenant une partie de bord supérieur s'étendant au-dessus de la lentille transparente,
- un harnais, gonflable, adapté pour s'étendre autour de la tête de l'utilisateur à l'opposé de la coque, afin de maintenir la coque sur le visage de l'utilisateur, le harnais comprenant au moins une partie postérieure destinée à venir en regard d'une partie arrière (occipitale) de la tête de l'utilisateur,
- un lien de guidage présentant une extrémité supérieure et une extrémité inférieure, l'extrémité supérieure étant liée à la partie de bord supérieur, et
- une plaque liée à l'extrémité inférieure du lien de guidage et présentant une face d'appui adaptée pour s'appliquer sur la coque, en regard de la cavité, la plaque étant liée à la partie postérieure du harnais.

La plaque et le lien de guidage permettent de positionner la partie postérieure du harnais et empêcher son introduction dans la (l'une des) cavité(s) du masque respiratoire. Ainsi, il n'est pas nécessaire de modifier la boite de rangement pour améliorer le rangement de l'équipement respiratoire dans la boite de rangement. En outre, les équipements respiratoires existant peuvent être aisément modifiés pour améliorer leur position de rangement.

Bien entendu, augmenter la taille de la plaque tend à réduire le risque d'introduction du harnais dans la cavité. Mais, plus la plaque est grande plus il est difficile de ranger l'équipement respiratoire dans la boite de rangement et de l'en ressortir rapidement sans l'endommager. Un compromis est donc nécessaire.

Selon un aspect supplémentaire, le lien de guidage présente de préférence une flexibilité en torsion inférieure à un demi-tour. Ainsi, le risque de vriller le harnais et de rangement inapproprié de l'équipement respiratoire est réduit.

Selon un autre aspect, le lien de guidage présente de préférence une forme allongée et plate. Ainsi, l'encombrement du lien de guidage lorsque le masque respiratoire est en position de rangement est réduit et le risque de rangement inapproprié de l'équipent respiratoire est réduit.

Selon un autre aspect, de préférence le lien de guidage présente une partie supérieure et une partie inférieure, le lien de guidage étant adapté pour être replié avec la partie supérieure sur la plaque et la partie inférieure entre la partie supérieure et la plaque. Ainsi, le positionnement du lien de guidage lorsque le masque respiratoire est en position de rangement est mieux contrôlé.

Selon un aspect complémentaire, la partie supérieure est de préférence rigide. Ainsi, le positionnement du lien de guidage lorsque le masque respiratoire est en position de rangement est encore mieux contrôlé. Il faut comprendre par rigide, le fait que le lien de guidage ne se plie pas sous son propre poids et le poids du harnais.

Selon un aspect encore complémentaire, la première partie présente de préférence une longueur supérieure ou égale à 10 centimètres, plus préférentiellement supérieure ou égale à 15 centimètres. Ainsi, le positionnement de la partie supérieure du lien de guidage est mieux contrôlé et le lien de guidage s'oppose plus efficacement à la présence d'une partie du harnais dans la cavité.

Selon un autre aspect, de préférence la partie supérieure comprend deux branches s'étendant longitudinalement et distantes transversalement (perpendiculairement à la direction dans laquelle s'étendent les branches) l'une de l'autre d'une distance supérieure ou égale à 3 centimètres, plus préférentiellement supérieure ou égale à 5 centimètres, et l'équipement respiratoire comprend deux parties basses s'étendant entre la partie supérieure et la plaque, autrement dit, la partie supérieure étant reliée à la plaque par l'intermédiaire de la partie inférieure.

Selon un autre aspect, la première partie est montée rotative sur la partie de bord supérieur. Ainsi, le positionnement du lien de guidage est bien contrôlé dans la position de rangement et son positionnement s'adapte à la morphologie de l'utilisateur.

Selon un autre aspect, la partie inférieure est au moins en partie souple et disposée entre la partie supérieure et la plaque. Ainsi, le lien de guidage s'adapte aisément lors du déploiement du harnais, lors de la mise en position de rangement de l'équipement respiratoire et en fonction de la morphologie de l'utilisateur. Il faut comprendre par souple le fait que la partie inférieure se plie sous son propre poids et le poids du harnais.

Selon un autre aspect, la partie inférieure comprend de préférence au moins un élément rigide. Ainsi, la torsion de la partie inférieure est réduite, tout en permettant de plier la partie inférieure.

Selon un aspect complémentaire, la deuxième partie comprend de préférence une succession d'éléments rigides articulés les uns par rapport aux autres. Ainsi, la partie inférieure peut se plier à de multiples endroits situés entre les éléments rigides.

Selon un autre aspect, de préférence la partie inférieure comprend une sangle, la sangle se prolongeant au-delà de l'extrémité inférieure en formant une boucle autour d'un tube du harnais et la boucle présente des extrémités maintenues sur la plaque. Ainsi, la sangle sert en outre à fixer la plaque au harnais.

Selon un aspect alternatif, de préférence l'extrémité inférieure du lien de guidage est liée à la partie postérieure du harnais par l'intermédiaire de la plaque.

Selon un autre aspect, de préférence l'équipement respiratoire est en outre muni d'une signalétique de bon positionnement dans une position de rangement. Ainsi, la mise en position de rangement satisfaisante de l'équipement respiratoire est facilitée et le risque que l'utilisateur mette l'équipement respiratoire dans une position de rangement incorrecte est réduit.

Selon un autre aspect, de préférence la coque comprend un couvre face oronasal et un support, le couvre face oronasal présentant une première cavité et étant adapté pour s'appliquer sur la tête de l'utilisateur autour de la bouche et du nez de l'utilisateur, le support supportant une lentille transparente et présentant une deuxième cavité.

Il est également proposé un ensemble respiratoire comprenant un équipement respiratoire précité et une boite de rangement comportant un logement adapté pour recevoir l'équipement respiratoire dans une position de rangement, ladite boite de rangement présentant une ouverture pour accéder au logement.

Il est en outre proposé un procédé pour ranger l'équipement respiratoire dans la boite de rangement de l'ensemble respiratoire précité, le procédé comprenant :
a) amener la plaque contre le couvre face oronasal,
b) faire pivoter le lien de guidage à proximité de son extrémité supérieure par rapport au masque respiratoire,
c) placer l'équipement respiratoire dans une position de rangement en repliant le lien de guidage de manière à superposer la partie supérieure du lien de guidage, la partie inférieure du lien de guidage et la plaque, et
d) introduire l'équipement respiratoire dans la boite de rangement.

### Brève description des figures

D'autres caractéristiques et avantages apparaîtront dans la description détaillée suivante, se référant aux dessins annexés dans lesquels :
- Fig. 1 représente un équipement respiratoire, en perspective de devant, dans une position d'utilisation,
- Fig. 2 représente l'équipement respiratoire, en perspective de derrière, dans la position d'utilisation, selon une première variante de réalisation,
- Fig. 3 représente l'équipement respiratoire, en perspective de derrière, dans une position de mise en place, selon une deuxième variante de réalisation,
- Fig. 4 représente à échelle agrandie une plaque de l'équipement respiratoire selon une troisième variante,
- Fig. 5 représente l'équipement respiratoire, dans une position de rangement, inséré dans une boite de rangement,
- Fig. 6 représente l'équipement respiratoire, dans une position inappropriée pour le rangement.

### Description détaillée

Les figures illustrent un ensemble respiratoire 1 comprenant un équipement respiratoire 100 et une boite de rangement 30. L'équipement respiratoire 100 est destiné à équiper un aéronef et à fournir un gaz respiratoire à un utilisateur en situation d'urgence ou à titre préventif. L'équipement respiratoire 100 comporte essentiellement un masque respiratoire 10, un harnais 20 gonflable, un lien de guidage 110 et une plaque occipitale 120.

Le masque respiratoire 10 comprend une coque. Dans le mode de réalisation illustré, la coque comprend un couvre face oronasal 14 et un support 17. Le couvre face oronasal 14 présente une première cavité 13 et un joint 16 s'étendant autour de la première cavité 13 et adapté pour s'appliquer sur la tête de l'utilisateur autour de la bouche et du nez de l'utilisateur. Le support 17 comprend une lentille transparente 18. Le support 17 présente une deuxième cavité 15. Le support 17 présente un joint 11 s'étendant autour de la deuxième cavité et destiné à s'appliquer sur la tête de l'utilisateur autour des yeux. Le support 17 comprend un contour 19 s'étendant autour de la lentille transparente 18. Le contour 19 présente une partie de bord supérieur 19a s'étendant au-dessus de la lentille transparente 18.

Dans le mode de réalisation illustré, le support 17 est amovible par rapport au couvre face oronasal 14 et forme une lunette amovible. La première cavité 13 peut être isolée de la deuxième cavité 15, notamment lorsque le support 17 est séparé du couvre face oronasal. En variante, le support 17 pourrait ne pas être libérable par rapport au couvre face oronasal 14 et, dans ce cas, la première cavité 13 et la deuxième cavité 15 peuvent ne pas être disjointes et former deux zones d'une cavité globale unique. Un tel masque respiratoire est usuellement dénommé full face.

Le masque respiratoire 10 comprend en outre un boitier de préhension 12. Le boitier de préhension 12 renferme un régulateur à la demande (non représenté) alimentant la première cavité 13 en un mélange respiratoire comprenant un gaz respiratoire et de l'air ambiant. Un tube d'alimentation 2 est fixé au boitier de préhension 12. Le tube d'alimentation 2 est relié en amont à une source de gaz respiratoire (non représentée) sous pression contenant de préférence au moins 95% d'oxygène et en aval au régulateur à la demande pour alimenter le régulateur à la demande en gaz respiratoire.

Le harnais 20 est adapté pour s'étendre autour de la tête de l'utilisateur à l'opposé de la coque, afin de maintenir la coque sur la tête de l'utilisateur, le harnais 20 comprenant une partie postérieure 25 destinée à venir en regard de la partie arrière (occipitale) de la tête de l'utilisateur. Dans le mode de réalisation illustré, le harnais 20 comprend un tube supérieur 21 et un tube inférieur 22 élastiquement extensible longitudinalement. Le tube supérieur 21 s'étend continûment entre une première extrémité supérieure 21a et une deuxième extrémité supérieure 21b maintenues sur le boitier de préhension 12. Le tube inférieur 22 s'étend continûment entre une première extrémité inférieure 22a et une deuxième extrémité inférieure 22b maintenues sur le boitier de préhension 12.

Le tube supérieur 21 est relié par l'une au moins parmi la première extrémité supérieure 21a et la deuxième extrémité supérieure 21b à une source de gaz sous pression par l'intermédiaire d'une vanne de gonflage (non représentée) permettant sélectivement d'introduire le gaz sous pression dans le tube supérieur 21 (afin d'augmenter la longueur du tube supérieur 21) ou d'évacuer le gaz sous pression (afin de contracter le tube supérieur 21) par retour élastique. Le tube inférieur 22 est relié par l'une au moins parmi la première extrémité inférieure 22a et la deuxième extrémité inférieure 22b à la source de gaz sous pression par l'intermédiaire de la vanne de gonflage (non représentée) permettant sélectivement d'introduire le gaz sous pression dans le tube inférieur 22 (afin d'augmenter la longueur du tube inférieur 22) ou d'évacuer le gaz sous pression (afin de contracter le tube inférieur 22) par retour élastique. Dans le mode de réalisation illustré, la source de gaz sous pression est constituée par la source de gaz respiratoire.

En alternative, le tube supérieur 21 et le tube inférieur 22 pourraient être remplacés par deux tubes latéraux reliés l'un à l'autre, de préférence par la plaque occipitale 120, les deux tubes latéraux seraient alors reliés au boitier de préhension pour l'un sensiblement au niveau de la première extrémité inférieure et la première extrémité supérieure (ou du contour 19) et pour l'autre sensiblement au niveau de la deuxième extrémité inférieure et la deuxième extrémité supérieure (ou du contour 19). Une telle alternative de harnais est bien connue.

Le lien de guidage 110 présente une extrémité supérieure 110a et une extrémité inférieure 110b. L'extrémité supérieure 110a est liée à la partie de bord supérieur 19a par l'intermédiaire d'un élément de liaison 111 fixé au bord supérieur 19a. L'extrémité inférieure 110b est liée à la partie postérieure 25 du harnais 20.

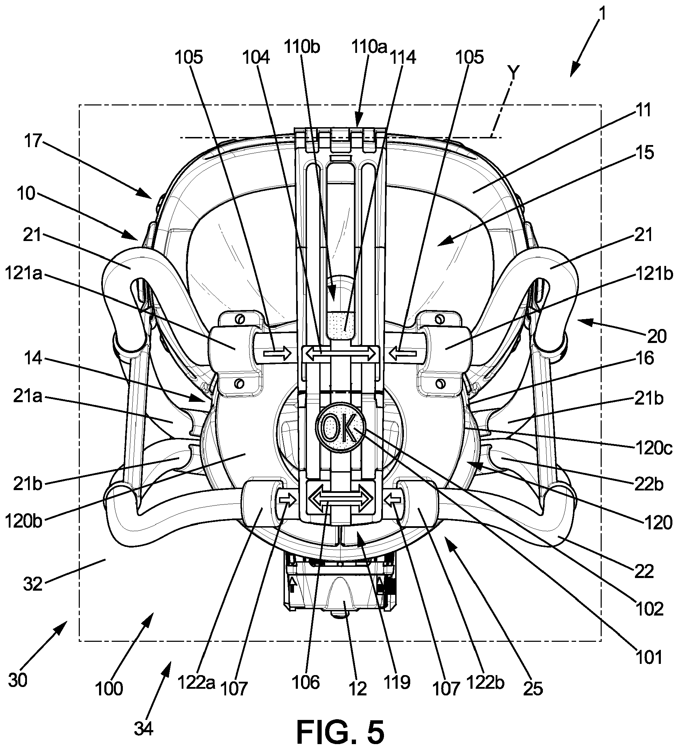

La plaque occipitale 120 présente une face interne 120a et une face externe 120b. La face interne 120a est adaptée pour s'appliquer sur le couvre face oronasal 14, en regard de la première cavité 13 dans une position de rangement de l'équipement respiratoire. La plaque occipitale 120 présente un contour 120c correspondant au contour du joint 16 du couvre face oronasal 14 contre lequel elle vient s'appliquer dans la position de rangement (voir en particulier FIG. 5), afin de guider l'opérateur sur le positionnement de la plaque occipitale 120. L'extrémité inférieure 110b du lien de guidage 110 est liée à la plaque occipitale 120. Dans le mode de réalisation illustré, quatre attaches 130 sont prévus pour la fixation de l'extrémité inférieure 110b du lien de guidage à la plaque occipitale 120. De préférence, l'extrémité inférieure 110b du lien de guidage 110 est fixée à la face interne 120a de la plaque occipitale 120. Dans le mode de réalisation illustré, un creux 124 formant rainure est prévu dans la face interne 120a de la plaque occipitale 120, de sorte que l'extrémité de basse 110b du lien de guidage 110 est noyée dans le creux.

La plaque occipitale 120 est munie d'éléments de maintien formant passages de tube. Dans le mode de réalisation illustré, la plaque occipitale comprend un premier élément de maintien supérieur 121a, un deuxième élément de maintien supérieur 121b, un premier élément de maintien inférieur 122a et un deuxième élément de maintien inférieur 122b. Le premier élément de maintien supérieur 121a, le deuxième élément de maintien supérieur 121b, le premier élément de maintien inférieur 122a et le deuxième élément de maintien inférieur 122b font saillie sur la face externe 120b de la plaque occipitale 120. Le premier élément de maintien supérieur 121a présente un alésage recevant une première partie intermédiaire supérieure 21c du tube supérieur 21. Le deuxième élément de maintien supérieur 121b présente un alésage recevant une deuxième partie intermédiaire supérieure 21d du tube supérieur 21. Le premier élément de maintien inférieur 122a présente un alésage recevant une première partie intermédiaire inférieure 22c du tube inférieur 22. Le deuxième élément de maintien inférieur 122b présente un alésage recevant une deuxième partie intermédiaire inférieure 22d du tube inférieur 22.

Le tube supérieur 21 comprend une portion arrière supérieure 21e s'étendant entre la première partie intermédiaire supérieure 21c et la deuxième partie intermédiaire supérieure 21d. Le tube inférieur 22 comprend une portion arrière inférieure 22e s'étendant entre la première partie intermédiaire inférieure 21c et la deuxième partie intermédiaire inférieure 21d.

Dans le mode de réalisation illustré, la partie postérieure 25 du harnais 20 comprend la première partie intermédiaire supérieure 21c, la partie arrière supérieure 21e, la deuxième partie intermédiaire supérieure 21d, la première partie intermédiaire inférieure 22c, la partie arrière inférieure 22e et la deuxième partie intermédiaire inférieure 22d.

En variante, la portion arrière supérieure 21e et/ou la portion arrière inférieure 22e pourrait être omise. Ainsi, le tube supérieur 21 (respectivement le tube inférieure 22) comprendrait deux portions disjointes reliées par la plaque occipitale 120, l'une des portions disjointes s'étendant entre la première extrémité supérieure 21a (respectivement la première extrémité inférieure 22a) et la première partie intermédiaire supérieure 21c (respectivement la première partie intermédiaire inférieure 22c) formant extrémité, l'autre des portions disjointes s'étendant entre la deuxième extrémité supérieure 21b (respectivement la deuxième extrémité inférieure 22b) et la deuxième partie intermédiaire supérieure 21d (respectivement la deuxième partie intermédiaire inférieure 22d) formant extrémité.

Selon une autre variante, la plaque occipitale 20 pourrait comprendre un unique élément de maintien supérieur à la place du premier élément de maintien supérieur 121a et du deuxième élément de maintien supérieur 121b et/ou un unique élément de maintien inférieur à la place du premier élément de maintien inférieur 122a et du deuxième élément de maintien inférieur 122b.

La plaque occipitale 120 reliant ainsi le tube supérieur 21 au tube inférieur 22 permet de maintenir l'espacement entre le tube supérieur 21 et le tube inférieur 22 dans la partie postérieure 25 du harnais et remplace les entretoises s'étendant habituellement entre le tube supérieur et le tube inférieur du harnais.

La plaque occipitale 120 est suffisamment rigide pour éviter qu'elle ne soit entrée en force dans la première cavité 13 du couvre face oronasal 14. La face interne 120a plaque occipitale 120 doit toutefois être suffisamment souple pour éviter de heurter la partie arrière de la tête de l'utilisateur. En particulier, pour éviter de créer une douleur au niveau de la partie occipitale de la tête de l'utilisateur, la plaque 120 présente un trou central 125. En outre, la face interne 120a de la plaque est de préférence recouverte au moins en partie d'un matériau souple.

Le lien de guidage 110 comprend une partie supérieure 112 et une partie inférieure 114. La partie supérieure 112 est reliée au niveau de l'extrémité supérieure 110a au bord supérieur 19a du support 17 par l'intermédiaire de l'élément de liaison 111. La partie supérieure 112 est monté mobile en rotation sur l'élément de liaison 111 autour d'un axe d'articulation Y s'étendant suivant une direction transversale, autrement dit sensiblement suivant le bord supérieur 19a. La partie inférieure 114 forme un lien souple et non élastique. La partie inférieure 114 est fixée à la partie supérieure 112, par des rivets, des boulons ou tout autre élément de fixation mécanique, au niveau d'une zone de jonction 119. La partie supérieure 112 s'étend de l'extrémité supérieure 110a à la zone de jonction 119. La partie inférieure 114 s'étend à la suite de la partie supérieure 112, de la zone de jonction 119 à l'extrémité inférieure 110b. La partie inférieure 114 relie la partie haute 112 à la plaque occipitale 120. La partie supérieure 112 est de préférence réalisée dans un matériau ayant une dureté modérée, de préférence une dureté shore de 90 minimum et/ou en silicone.

Le lien de guidage 100 permet de réduire le risque que la plaque occipitale 120 soit rangée trop haut par rapport au masque respiratoire 10 et puisse buter contre le support 17 lors du gonflage du harnais 20, ce qui entrainerait une augmentation du temps nécessaire au gonflage du harnais 20, voire un déploiement insatisfaisant du harnais 20 au cours du gonflage.

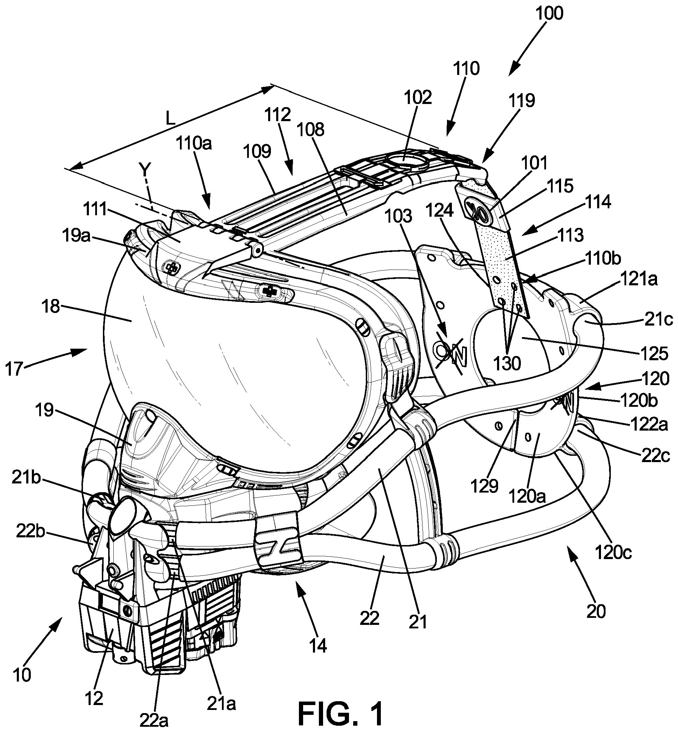

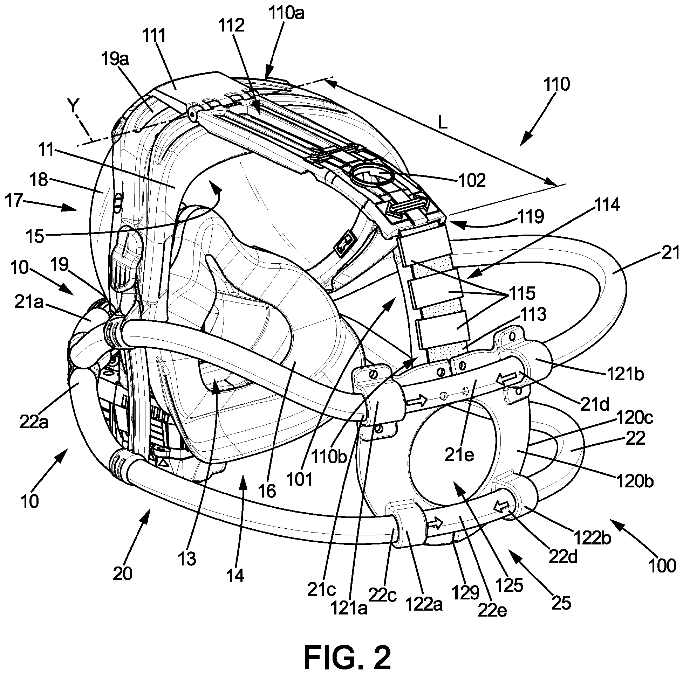

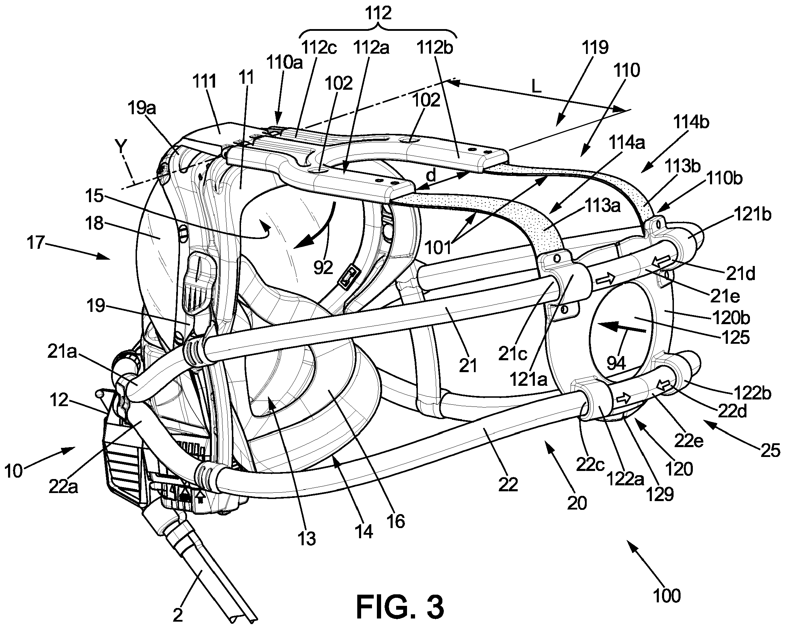

En position d'utilisation de l'équipement respiratoire 100, illustré aux figures 1 et 2, l'équipement respiratoire est destiné à être positionné sur la tête d'un utilisateur (non représentée) avec le harnais 20 déplié mais non gonflé, la partie supérieure 112 s'étendant sensiblement horizontalement autour de la tête de l'utilisateur. Dans le mode de réalisation illustré aux figures 1 et 2, la partie supérieure 112 du lien de guidage 110 s'incurve progressivement à proximité de la zone de jonction 119, afin de mieux suivre la forme de la tête. La partie inférieure 114 présente alors une forme courbe s'étendant essentiellement sur l'arrière de la tête de l'utilisateur. La partie inférieure 114 s'étend sensiblement verticalement derrière la tête de l'utilisateur, à proximité de l'extrémité inférieure 110b et en partie sur le dessus de la tête, sensiblement horizontalement, à proximité de la zone de jonction 119. En variante, la partie supérieure 112 pourrait être entièrement plane (de l'extrémité supérieure 110a à la zone de jonction 119, tel qu'illustré à la figure 3).

En position de mise en place de l'équipement respiratoire, illustrée à la figure 3, le harnais 20 est déployé, gonflé. La partie supérieure 112 du lien de guidage 110 est sensiblement dans la même position qu'en position d'utilisation de l'équipement respiratoire 100. En revanche, la forme de la partie inférieure 114 du lien de guidage 110 est modifiée lors du gonflement du harnais 20 (inversement lors du dégonflement du harnais 20), la partie inférieure 114 du lien de guidage 110 s'étendant essentiellement horizontalement lorsque le harnais 20 est gonflé, la partie inférieure 114 du lien de guidage 110 ne s'étendant sensiblement verticalement qu'à proximité immédiate de l'extrémité inférieure 110b. Du fait que la partie inférieure 114 est au moins en partie souple, la partie inférieure 114 s'adapte aisément au déplacement de la partie postérieure 25 du harnais 20 à l'opposé (en éloignement) du masque respiratoire 10 lors du gonflage (induisant une élongation) du premier tube 21 et du deuxième tube 22.

En position de rangement de l'équipement respiratoire 110, illustrée à la figure 5, la plaque occipitale 120 est en appui contre la coque. Plus précisément, la plaque occipitale est en appui sur le couvre face oronasal 14. De préférence concomitamment au rapprochement de la plaque occipitale 120 du couvre face oronasal 14 tel qu'indiqué schématiquement par la flèche 92 à la figure 3, l'utilisateur fait pivoter la partie supérieure 112 du lien de guidage 110 par rapport au masque respiratoire 10 par rotation autour de l'axe d'articulation Y d'environ 90 degrés, tel qu'indiqué schématiquement par la flèche 94 à la figure 3, jusqu'à amener la partie supérieure 112 du lien de guidage 110 en appui sur la plaque occipitale 120.

En position de rangement, la partie inférieure 114 est repliée (à 180 degrés) à proximité de la zone de jonction 19 et à proximité de l'extrémité inférieure 110b. La partie inférieure 114 s'étend entre la partie supérieure 112 et la plaque occipitale 120 entre ces deux repliements. Dans le mode de réalisation illustré aux figures 1 et 2, la partie supérieure 112 présente deux rebords latéraux 108, 109 entre lesquels la partie inférieure 114 est maintenue dans la position de rangement.

La partie haute 112 présente une longueur L (voir en particulier les figures 1 à 3), perpendiculairement à l'axe d'articulation Y, d'au moins 10 centimètres, de préférence au moins 15 centimètres et plus préférentiellement environ 17 centimètres, pour éviter que l'extrémité libre de la partie haute vienne se coincer dans la partie inférieure du joint 16 du couvre face oronasal 14.

L'équipement respiratoire est en outre muni d'une signalétique de bon positionnement en position de rangement. Ainsi, la partie inférieure 114 du lien de guidage 110 est en outre munie d'un indicateur de bon positionnement 101, constitué dans le mode de réalisation d'un repère vert et de préférence d'une mention "OK", tandis que la partie supérieure 112 du lien de guidage 110 comprend un orifice de visualisation 102. Tel qu'illustré à la figure 5, lorsque l'équipement respiratoire 110 est en position de rangement satisfaisante, l'indicateur de bon positionnement 101 est en regard de l'orifice de visualisation 102 de la partie haute 102 et donc visible par l'utilisateur. En cas de positionnement incorrect de la plaque occipitale 120, la partie inférieure 114 se retrouve décalé par rapport à la partie supérieure 112 du lien de guidage 110, en particulier l'indicateur de bon positionnement 101 ne se trouve plus en regard de l'orifice de visualisation 102. L'indicateur de bon positionnement étant derrière la partie supérieure 112 du lien de guidage 110, l'indicateur de bon positionnement n'est alors pas visible.

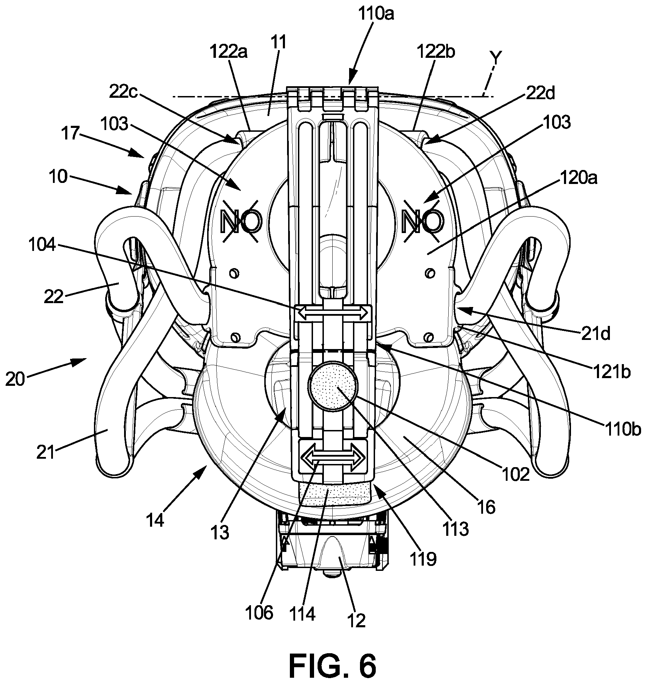

Ainsi, la plaque occipitale 120 est munie d'un repère visuel indiquant à l'opérateur si la plaque occipitale 120 est positionnée correctement en position de rangement, principalement pour éviter le retournement de la plaque de 180° quel que soit la direction de retournement. En outre, tel qu'illustré à la figure 6, l'équipement respiratoire 110 porte un indicateur de mauvais positionnement 103. Dans le mode de réalisation illustré, l'indicateur de mauvais positionnement 103 est constituée par des indications "NO" ou pictogrammes équivalents (non représentés) portés sur la face interne 120a de la plaque occipitale 120, de préférence avec une signalétique correspondant à un danger, telle que la couleur rouge et/ou un triangle, ou une croix symbolisant que la plaque occipitale 120 n'est pas positionné sur la bonne face. Cet indicateur de mauvais positionnement 103 étant porté sur la face interne 120a de la plaque occipitale 120, il n'est visible par l'utilisateur dans la position de rangement que si la plaque occipitale 120 est positionnée, retournée à 180 degrés (sens dessus dessous, voire latéralement), signe d'un mauvais rangement du harnais 20. En effet, la face interne 120a est destinée à être en regard de la coque 14, 17 et à venir au contact de la coque lorsque l'équipement respiratoire 100 est dans la position de rangement correcte, en particulier en appui sur le couvre face oronasal 14 dans le mode de réalisation illustré. Dans la configuration illustrée à la figure 6, la face interne 120a est tournée à l'opposé du masque respiratoire 10, ce qui induit un mauvais positionnement du harnais 20, et est alors visible par l'utilisateur.

De plus, la partie supérieure 112 du lien de guidage 110 et la plaque occipitale 120 sont munies de marquages (des flèches dans le mode de réalisation illustré, de préférence blanches ou vertes) qui vont guider l'utilisateur sur l'alignement et le bon positionnement à respecter. Plus précisément, la partie supérieure 112 du lien de guidage 110 présente un premier repère 104 de positionnement du tube supérieur 21 et un deuxième repère 106 de positionnement du tube inférieur 22. Le premier repère 104 et le deuxième repère 106 sont constitués par des doubles flèches dans le mode de réalisation illustré. Le tube supérieur 21 présente des indicateurs supérieurs 105 de la position du tube supérieur 21 et le tube inférieur 22 présente des indicateurs inférieurs 107 de la position du tube inférieur 22. Les indicateurs supérieurs 105 de la position du tube supérieur 21 et les indicateurs inférieurs 107 de la position du tube inférieur 22 sont constitués par des flèches dans le mode de réalisation illustré. Lorsque l'ensemble respiratoire 110 est dans la position de rangement correcte, telle que représentée à la figure 5, les indicateurs supérieurs 105 de la position du tube supérieur 21 sont situés de part et d'autre du premier repère 104 et au même niveau que le premier repère 104, de sorte que les indicateurs supérieurs 105 de la position du tube supérieur 21 et le premier repère 104 sont alignés. En outre, les indicateurs inférieurs 107 de la position du tube inférieur 107 sont situés de part et d'autre du deuxième repère 106 et au même niveau que le deuxième repère 106, de sorte que les indicateurs inférieurs 107 de la position du tube inférieur 107 et le deuxième repère 106 sont alignés.

La boite de rangement 30 comporte un logement 32 et une ouverture 34 d'accès au logement 32. Lorsque l'équipement respiratoire 100 est dans la position de rangement correct, l'utilisateur introduit l'équipement respiratoire 100 dans le logement 32 par l'ouverture 34 de la boite rangement 30 en tenant l'équipement respiratoire 100 par le boitier de préhension 12. Des portes peuvent être prévues pour obturer au moins partiellement l'ouverture 34, tout en permettant de saisir le boitier de préhension 12.

Lorsque l'utilisateur souhaite placer l'équipement respiratoire 110 sur sa tête, il saisit l'équipement respiratoire 100 par le boitier de préhension 12. Puis, il tire sur le boitier de préhension 12 pour extraire l'équipement respiratoire 100 du logement 32 de la boite de rangement 30, il actionne une commande agissant sur la vanne de gonflage pour gonfler le tube supérieur 21 et le tube inférieur 22 du harnais 20. Le harnais 20 se déploie en une fraction de seconde. L'utilisateur place l'équipement respiratoire 100 au-dessus de sa tête, puis descend l'équipement respiratoire 100 autour de sa tête. Enfin, l'utilisateur relâche la commande agissant sur la vanne de gonflage, le tube supérieur 21 et le tube inférieur 22 se dégonflent, le harnais 20 se contracte et le masque respiratoire 10 est appliqué sur le visage de l'utilisateur, tandis que la plaque occipitale 120 est appliquée sur l'arrière de la tête de l'utilisateur.

Des ressorts peuvent être disposés entre l'élément de liaison 111 et la partie supérieure 112 du lien de guidage, pour solliciter la remontée de la partie supérieure 112 du lien de guidage 110 dans une position sensiblement horizontale (perpendiculaire au support 17), lors du gonflage du harnais 20 provoquant son déploiement depuis la position de rangement.

Afin de gagner en souplesse et optimiser l'épaisseur globale lors du rangement, la plaque occipitale 120 est de préférence réalisée en 2 parties, dénommées partie gauche 126 et partie droite 128, juxtaposées suivant la direction transversale. Les deux parties peuvent être reliées par un élément de jonction 129 comprenant en particulier une charnière ou un élément souple surmoulé (silicone, sangle, lame ressort). L'amplitude de rotation entre la partie gauche 126 et la partie droite 128 est de préférence inférieure à 30 degrés, (de préférence environ 20 degrés) dans les deux sens, soit une amplitude maximale de 60 degrés, de préférence 40 degrés, afin d'éviter que la partie gauche 126 et la partie droite 128 puissent être repliées et que la plaque occipitale 120 risque d'être insérée dans la première cavité 13 du couvre face oronasal 14 ou la deuxième cavité 15 du support 17.

Cette flexion entre la partie gauche 126 et la partie droite 128 de la plaque occipitale 120 améliore le confort de l'utilisateur en épousant au plus près le galbe du crâne de l'utilisateur.

Dans les modes de réalisation illustrés, la partie supérieure 112 et la partie inférieure 114 du lien de guidage 110 présentent une forme allongée et plate. En outre, la partie inférieure 114 comprend avantageusement une sangle 113.

Dans le mode de réalisation illustré à la figure 1, la partie inférieure 114 du lien de guidage 110 comprend en outre une plaquette rigide 115. La plaquette 115 est de préférence fixée sur la sangle 113. La plaquette 115 limite la possibilité de torsion de la partie inférieure 114 du lien de guidage 110, ce qui réduit le risque de mal positionner la plaque occipitale 20 en la retournant de 180 degrés par torsion de la partie inférieure 114 du lien de guidage 110. La torsion de la partie inférieure 114 du lien de guidage 110 est limitée par la plaquette 115, rigide, qui ne permet une torsion de la sangle 113 qu'à l'écart de la plaquette 115, sans limiter la flexion de la sangle 113 à l'écart de la plaquette 115, de sorte qu'en positionnement correctement la plaquette (à l'écart des zones où la partie inférieure 114 doit être repliée) la plaquette 115 ne nuit pas à la flexion nécessaire pour que le lien de guidage 110 s'adapte au passage de l'équipement respiratoire 100 de la position d'utilisation à la position de rangement ou inversement. L'indicateur de bon positionnement 101 est de préférence porté par la plaquette 115.

La variante illustrée à la figure 2 se distingue essentiellement du mode de réalisation illustré à la figure 1 en ce que la partie inférieure 114 du lien de guidage 110 comprend trois plaquettes rigides 115 réparties sur la sangle 113. Seule la plaquette rigide 115 disposée entre les deux autres porte l'indicateur de bon positionnement 101. Selon une autre variante (non représentée), la partie inférieure 114 du lien de guidage 110 comprend cinq plaquettes rigides 115. Avantageusement, la partie inférieure 114 du lien de guidage 110 comprend entre une et cinq plaquettes rigides 115, une seule portant l'indicateur de bon positionnement 101 puisque le bon positionnement est unique. Les plaquettes rigides 115 sont de préférence surmoulées sur la sangle 113 ou fixées par clipsage, rivetage boulonnage ou autre moyen de fixation approprié. Le nombre et la forme des plaquettes est adapté pour éviter le risque de créer une douleur à l'utilisateur du fait du contact avec le crâne de l'utilisateur.

La variante illustrée à la figure 3 se distingue essentiellement du mode de réalisation illustré à la figure 1 en ce que la partie inférieure 114 du lien de guidage 110 est dépourvue de plaquette rigide, de sorte que l'indicateur de bon positionnement est porté directement par la sangle 113.

La variante représentée à la figure 3, se distingue en outre essentiellement du mode de réalisation illustré à la figure 1 (et indépendamment du nombre de plaquette 115 que comprend la partie inférieure 114 du lien de guidage 110) par le fait que la partie supérieure 112 du lien de guidage 100 comprend une première branche 112a et une deuxième branche 112b au niveau de la zone de jonction 119 et le lien de guidage 110 comprend une première partie inférieure 114a et une deuxième partie inférieure 114b distinctes, reliant la partie supérieure 112 à la plaque occipitale 120. La première branche 112a est distante de la deuxième branche 112b d'une distance d supérieure ou égale à 3 centimètres, de préférence au moins égale à 5 centimètres. La première partie inférieure 114a comprend une première sangle 113a et la deuxième partie inférieure 114b comprend une deuxième sangle 113b. Ainsi, la facilité de positionnement correct de l'équipement respiratoire dans la position de rangement et la compacité de l'équipement respiratoire dans la position de rangement pourront être améliorés. En outre, cela offre la possibilité de faire passer une queue de cheval ou un chignon.

Dans le mode de réalisation illustré à la figure 3, la partie supérieure 112 comprend en outre une base 112c s'étendant depuis l'extrémité supérieure 110a, la base 112c étant reliée à l'opposé de l'extrémité supérieure 110a à la première branche 112a et à la deuxième branche 112b. La partie supérieure 112 est donc articulée par rapport à l'élément de liaison 111 par la base 112c.

En variante, la partie supérieure 112 pourrait être dépourvue de la base 112c, chacune des première branche 112a et deuxième branche 112b s'étendant jusqu'à l'extrémité supérieure 110a et étant directement articulée sur l'élément de liaison 111.

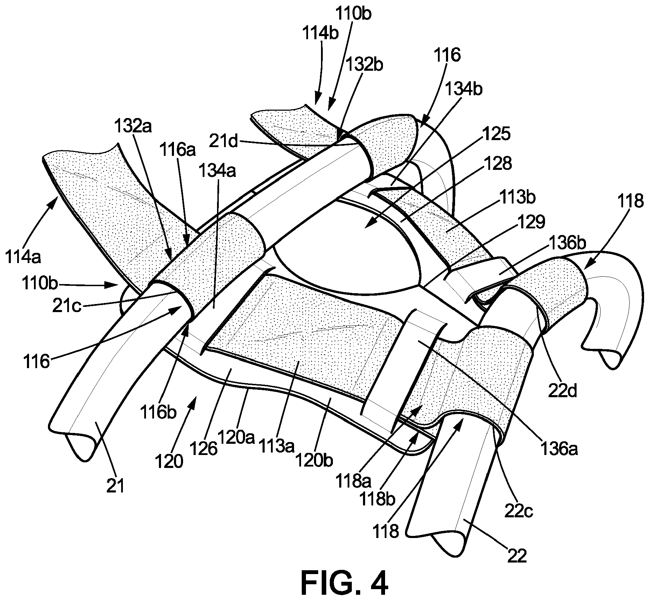

La variante représentée à la figure 4 se distingue essentiellement de la variante représentée à la figure 3 en ce que la première sangle 113a et la deuxième sangle 113b se prolongent au-delà de l'extrémité inférieure 110b, en formant chacune une boucle proximale 116 et une boucle distale 118, et la plaque occipitale 120 est dépourvue du premier élément de maintien supérieur 121a, du premier élément de maintien inférieur 122a, du deuxième élément de maintien supérieur 121b et du deuxième élément de maintien inférieur 122b.

La boucle proximale 116 de la première sangle 113a et de la deuxième sangle 113b s'étend entre une première extrémité proximale 116a et une deuxième extrémité proximale 116b autour respectivement de la première partie intermédiaire supérieure 21c et de la deuxième partie intermédiaire supérieure 21d du tube supérieur 21. La boucle distale 118 de la première sangle 113a et de la deuxième sangle 113b s'étend entre une première extrémité distale 118a et une deuxième extrémité distale 118b autour respectivement de la première partie intermédiaire inférieure 22c et de la deuxième partie intermédiaire inférieure 22d du tube inférieur 22. La première extrémité distale 118a et la deuxième extrémité distale 118b de la première sangle 113a et de la deuxième sangle sont solidarisées par couture, soudure ou analogue.

La première sangle 113a et la deuxième sangle 113b passent alternativement sur la face interne 120a et la face externe 120b de la plaque 120. La plaque occipitale 120 comprend un premier passant supérieur 132a, un premier passant intermédiaire 134a, un premier passant inférieur 136a, un deuxième passant supérieur 132b, un deuxième passant intermédiaire 134b, un deuxième passant inférieur 136b. La première sangle 113a est au contact de la face interne 120a de la plaque occipitale 120 au niveau du premier passant supérieur 132a, du premier passant intermédiaire 134a et du premier passant inférieur 136a. La première sangle 113a est au contact de la face externe 120b de la plaque occipitale 120 de part et d'autre du premier passant supérieur 132a, du premier passant intermédiaire 134a et du premier passant inférieur 136a, si ce n'est qu'entre le premier passant supérieur 132a et le premier passant intermédiaire 134a le tube supérieur 21 est interposé entre la face externe 120b de la plaque occipitale 120 et la première sangle 113a. La deuxième sangle 113b est au contact de la face interne 120a de la plaque occipitale 120 au niveau du deuxième passant supérieur 132b, du deuxième passant intermédiaire 134b et du deuxième passant inférieur 136b. La deuxième sangle 113b est au contact de la face externe 120b de la plaque occipitale 120 de part et d'autre du deuxième passant supérieur 132b, du deuxième passant intermédiaire 134b et du deuxième passant inférieur 136b, si ce n'est qu'entre le deuxième passant supérieur 132b et le deuxième passant intermédiaire 134b le tube supérieur 21 est interposé entre la face externe 120b de la plaque occipitale 120 et la deuxième sangle 113b.

Le premier passant supérieur 132a est situé à la première extrémité proximale 116a de la première sangle 113a, le premier passant intermédiaire 134a est situé à la deuxième extrémité proximale 116b de la première sangle 113a et le premier passant inférieur 136a est situé à la première extrémité distale 118a et à la deuxième extrémité distale 118b de la première sangle 113a. Le deuxième passant supérieur 132b est situé à la première extrémité proximale 116a de la deuxième sangle 113b, le deuxième passant intermédiaire 134b est situé à la deuxième extrémité proximale 116b de la deuxième sangle 113a et le deuxième passant inférieur 136b est situé à la première extrémité distale 118a et à la deuxième extrémité distale 118b de la deuxième sangle 113b.

Bien entendu l'invention n'est nullement limitée au(x) mode(s) de réalisation décrit(s) à titre illustratif, non limitatif. Ainsi, la plaque occipitale 20 pourrait être ajourée, former une grille ou analogue, la plaque occipitale 20 ayant essentiellement pour fonction de s'opposer à l'introduction d'une partie du harnais 20 dans la première cavité 13 du couvre face oronasal 14 ou la deuxième cavité 15 du support 17.

## Revendications

1. Equipement respiratoire (100) pour aéronef, l'équipement respiratoire étant destiné à fournir un gaz respiratoire à un utilisateur et comportant :
- un masque respiratoire (10) comprenant une coque (14, 17), la coque (14, 17) présente une cavité (13, 15) et est adaptée pour s'appliquer sur la tête de l'utilisateur autour de la bouche et du nez de l'utilisateur, la coque (14, 17) supportant une lentille transparente (18) et comprenant une partie de bord supérieur (19a) s'étendant au-dessus de la lentille transparente (18),
- un harnais (20), gonflable, adapté pour s'étendre autour de la tête de l'utilisateur à l'opposé de la coque, afin de maintenir la coque sur la tête de l'utilisateur, le harnais (20) comprenant au moins une partie postérieure (25) destinée à venir en regard d'une partie occipitale de la tête de l'utilisateur,
- un lien de guidage (110) présentant une extrémité supérieure (110a) et une extrémité inférieure (110b), l'extrémité supérieure (110a) étant liée à la partie de bord supérieur (19a), et
- une plaque (120) liée à l'extrémité inférieure (110b) du lien de guidage (110) et présentant une face d'appui (120a) adaptée pour s'appliquer sur la coque (14, 17), en regard de la cavité (13, 15), la plaque (120) étant liée à la partie postérieure (25) du harnais (20),
dans lequel :
- le lien de guidage présente une partie supérieure (112) et une partie inférieure (114), la partie supérieure (112) étant rigide l'équipement étant **caractérisé en ce que**: - le lien de guidage (110) est adapté pour être replié avec la partie supérieure (112) sur la plaque (120) et la partie inférieure (114) entre la partie supérieure (112) et la plaque (120),
- la partie supérieure (112) présente une longueur (L) supérieure ou égale à 10 centimètres,
- la partie supérieure (112) est montée rotative sur la partie de bord supérieur (19a), et
- la partie inférieure (114) est au moins en partie souple et disposée entre la partie supérieure (112) et la plaque (120).

2. Equipement respiratoire selon la revendication 1 dans lequel le lien de guidage (110) présente une flexibilité en torsion inférieure à un demi-tour.

3. Equipement respiratoire selon l'une quelconque des revendications précédentes dans lequel le lien de guidage (110) présente une forme allongée et plate.

4. Equipement respiratoire selon l'une quelconque des revendications précédentes dans lequel la partie supérieure (112) comprend deux branches (112a, 112b) s'étendant longitudinalement et distantes latéralement l'une de l'autre d'une distance (d) supérieure ou égale à 3 centimètres et l'équipement respiratoire comprend deux parties basses (114a, 114b) s'étendant entre la partie supérieure (112) et la plaque (120).

5. Equipement respiratoire selon la revendication précédente dans lequel la partie inférieure (114) comprend au moins un élément rigide (115).

6. Equipement respiratoire selon la revendication précédente dans lequel la partie inférieure comprend une succession d'éléments rigides (115) articulés les uns par rapport aux autres.

7. Equipement respiratoire selon l'une quelconque des revendications précédentes dans lequel la partie inférieure (114) comprend une sangle, la sangle se prolongeant au-delà de l'extrémité inférieure (110b) en formant une boucle (116) autour d'un tube (21, 22) du harnais (20) et la boucle (116) présente des extrémités (116a, 116b) maintenues sur la plaque (120).

8. Equipement respiratoire selon l'une quelconque des revendications 1 à 6 dans lequel l'extrémité inférieure (110b) du lien de guidage (110) est liée à la partie postérieure (25) du harnais (20) par l'intermédiaire de la plaque (120).

9. Equipement respiratoire selon l'une quelconque des revendications précédentes dans lequel l'équipement respiratoire (110) est en outre muni d'une signalétique de bon positionnement (101, 102, 104, 105, 106, 107) dans une position de rangement.

10. Equipement respiratoire selon l'une quelconque des revendications précédentes dans lequel la coque (14, 17) comprend un couvre face oronasal (14) et un support (17), le couvre face oronasal (14) présentant une première cavité (13) et étant adapté pour s'appliquer sur la tête de l'utilisateur autour de la bouche et du nez de l'utilisateur, le support (17) supportant une lentille transparente (18) et présentant une deuxième cavité (15).

11. Ensemble respiratoire (1) comprenant un équipement respiratoire selon l'une quelconque des revendications précédentes et une boite de rangement (30) comportant un logement (32) adapté pour recevoir l'équipement respiratoire (100) dans une position de rangement, ladite boite de rangement (30) présentant une ouverture (34) pour accéder au logement (32).

12. Procédé pour ranger un équipement respiratoire (1) pour aéronef dans une boite de rangement (30), dans lequel :
l'équipement respiratoire (100) et la boite de rangement (30) sont compris dans un ensemble respiratoire (1), la boite de rangement (30) comporte un logement (32) adapté pour recevoir l'équipement respiratoire (100) dans une position de rangement, ladite boite de rangement (30) présentant une ouverture (34) pour accéder au logement (32),
l'équipement respiratoire est destiné à fournir un gaz respiratoire à un utilisateur et comporte :
- un masque respiratoire (10) comprenant une coque (14, 17), la coque (14, 17) présente une cavité (13, 15) et est adaptée pour s'appliquer sur la tête de l'utilisateur autour de la bouche et du nez de l'utilisateur, la coque (14, 17) supportant une lentille transparente (18) et comprenant une partie de bord supérieur (19a) s'étendant au-dessus de la lentille transparente (18),
- un harnais (20), gonflable, adapté pour s'étendre autour de la tête de l'utilisateur à l'opposé de la coque, afin de maintenir la coque sur la tête de l'utilisateur, le harnais (20) comprenant au moins une partie postérieure (25) destinée à venir en regard d'une partie occipitale de la tête de l'utilisateur,
- un lien de guidage (110) présentant une extrémité supérieure (110a) et une extrémité inférieure (110b), l'extrémité supérieure (110a) étant liée à la partie de bord supérieur (19a), et
- une plaque (120) liée à l'extrémité inférieure (110b) du lien de guidage (110) et présentant une face d'appui (120a) adaptée pour s'appliquer sur la coque (14, 17), en regard de la cavité (13, 15), la plaque (120) étant liée à la partie postérieure (25) du harnais (20),
dans lequel :
- le lien de guidage présente une partie supérieure (112) et une partie inférieure (114), la partie supérieure (112) étant rigide et le lien de guidage (110) étant adapté pour être replié avec la partie supérieure (112) sur la plaque (120) et la partie inférieure (114) entre la partie supérieure (112) et la plaque (120),
le procédé étant **caractérisé en ce qu'**il comprend :
a) amener la plaque (120) contre la coque (14, 17),
b) faire pivoter le lien de guidage (110) à proximité de son extrémité supérieure (110a) par rapport au masque respiratoire (10),
c) placer l'équipement respiratoire dans une position de rangement en repliant le lien de guidage (110) de manière à superposer la partie supérieure (112) du lien de guidage (110), la partie inférieure (114) du lien de guidage (110) et la plaque (120), et
d) introduire l'équipement respiratoire dans la boite de rangement.

## Patentansprüche

1. Atemausrüstung (100) für Luftfahrzeuge, wobei die Atemausrüstung dazu bestimmt ist, einem Benutzer ein Atemgas bereitzustellen, enthaltend:
- eine Atemmaske (10) mit einer Schale (14, 17), wobei die Schale (14, 17) einen Hohlraum (13, 15) aufweist und dazu ausgelegt ist, am Kopf des Benutzers um Mund und Nase des Benutzers herum anzuliegen, wobei die Schale (14, 17) eine transparente Linse (18) trägt und einen oberen Randabschnitt (19a) aufweist, der sich oberhalb der transparenten Linse (18) erstreckt,
- ein aufblasbares Gurtzeug (20), das dazu ausgelegt ist, sich der Schale entgegengesetzt um den Kopf des Benutzers herum zu erstrecken, um die Schale am Kopf des Benutzers zu halten, wobei das Gurtzeug (20) zumindest einen hinteren Abschnitt (25) umfasst, der dazu bestimmt ist, einem Hinterkopfbereich des Kopfes des Benutzers gegenüberzuliegen,
- ein Führungsband (110), das ein oberes Ende (110a) und ein unteres Ende (110b) aufweist, wobei das obere Ende (110a) mit dem oberen Randabschnitt (19a) verbunden ist, und
- eine Platte (120), die mit dem unteren Ende (110b) des Führungsbandes (110) verbunden ist und eine Auflagefläche (120a) aufweist, die dazu ausgelegt ist, dem Hohlraum (13, 15) gegenüberliegend an der Schale (14, 17) anzuliegen, wobei die Platte (120) mit dem hinteren Abschnitt (25) des Gurtzeugs (20) verbunden ist,
- wobei das Führungsband einen oberen Abschnitt (112) und einen unteren Abschnitt (114) aufweist, wobei der obere Abschnitt (112) starr ausgeführt ist,
- wobei die Ausrüstung **dadurch gekennzeichnet ist, dass**
- das Führungsband (110) dazu ausgelegt ist, mit dem oberen Abschnitt (112) auf die Platte (120) und mit dem unteren Abschnitt (114) zwischen dem oberen Abschnitt (112) und der Platte (120) umgelegt zu werden,
- der obere Abschnitt (112) eine Länge (L) größer oder gleich 10 Zentimeter aufweist,
- der obere Abschnitt (112) drehbar an dem oberen Randabschnitt (19a) gelagert ist, und
- der untere Abschnitt (114) zumindest teilweise nachgiebig ist und zwischen dem oberen Abschnitt (112) und der Platte (120) angeordnet ist.

2. Atemausrüstung nach Anspruch 1,
wobei das Führungsband (110) eine Torsionsflexibilität von weniger als einer halben Umdrehung aufweist.

3. Atemausrüstung nach einem der vorhergehenden Ansprüche,
wobei das Führungsband (110) eine längliche, flache Form aufweist.

4. Atemausrüstung nach einem der vorhergehenden Ansprüche,
wobei der obere Abschnitt (112) zwei Schenkel (112a, 112b) aufweist, die sich in Längsrichtung erstrecken und seitlich um einen Abstand (d) größer oder gleich 3 Zentimeter voneinander beabstandet sind, und
wobei die Atemausrüstung zwei untere Abschnitte (114a, 114b) aufweist, die sich zwischen dem oberen Abschnitt (112) und der Platte (120) erstrecken.

5. Atemausrüstung nach dem vorhergehenden Anspruch,
wobei der untere Abschnitt (114) mindestens ein starres Element (115) umfasst.

6. Atemausrüstung nach dem vorhergehenden Anspruch,
wobei der untere Abschnitt eine Aufeinanderfolge von starren Elementen (115) umfasst, die gelenkig miteinander verbunden sind.

7. Atemausrüstung nach einem der vorhergehenden Ansprüche,
wobei der untere Abschnitt (114) einen Gurt umfasst, wobei sich der Gurt über das untere Ende (110b) hinaus erstreckt, indem er eine Schlaufe (116) um einen Schlauch (21, 22) des Gurtzeugs (20) bildet, und die Schlaufe (116) Enden (116a, 116b) aufweist, die an der Platte (120) gehalten werden.

8. Atemausrüstung nach einem der Ansprüche 1 bis 6,
wobei das untere Ende (110b) des Führungsbandes (110) über die Platte (120) mit dem hinteren Abschnitt (25) des Gurtzeugs (20) verbunden ist.

9. Atemausrüstung nach einem der vorhergehenden Ansprüche,
wobei die Atemausrüstung (110) ferner mit einer Beschilderung (101, 102, 104, 105, 106, 107) für die richtige Positionierung in einer Verstauposition versehen ist.

10. Atemausrüstung nach einem der vorhergehenden Ansprüche,
wobei die Schale (14, 17) eine oronasale Gesichtsabdeckung (14) und einen Träger (17) umfasst, wobei die oronasale Gesichtsabdeckung (14) einen ersten Hohlraum (13) aufweist und dazu ausgelegt ist, am Kopf des Benutzers um Mund und Nase des Benutzers herum anzuliegen, wobei der Träger (17) eine transparente Linse (18) trägt und einen zweiten Hohlraum (15) aufweist.

11. Atemanordnung (1) mit einer Atemausrüstung nach einem der vorhergehenden Ansprüche und einer Aufbewahrungsbox (30) mit einem Gehäuse (32), das zur Aufnahme der Atemausrüstung (100) in einer Verstauposition ausgelegt ist, wobei die Aufbewahrungsbox (30) eine Öffnung (34) für den Zugriff auf das Gehäuse (32) aufweist.

12. Verfahren zum Verstauen einer Atemausrüstung (1) für Luftfahrzeuge in einer Aufbewahrungsbox (30), wobei
die Atemausrüstung (100) und die Aufbewahrungsbox (30) in einer Atemanordnung (1) enthalten sind, wobei die Aufbewahrungsbox (30) ein Gehäuse (32) enthält, das dazu ausgelegt ist, die Atemausrüstung (100) in einer Verstauposition aufzunehmen, wobei die Aufbewahrungsbox (30) eine Öffnung (34) für den Zugang zum Gehäuse (32) aufweist,
wobei die Atemausrüstung dazu bestimmt ist, einem Benutzer ein Atemgas bereitzustellen und enthält:
- eine Atemmaske (10) mit einer Schale (14, 17), wobei die Schale (14, 17) einen Hohlraum (13, 15) aufweist und dazu ausgelegt ist, am Kopf des Benutzers um Mund und Nase des Benutzers herum anzuliegen, wobei die Schale (14, 17) eine transparente Linse (18) trägt und einen oberen Randabschnitt (19a) aufweist, der sich oberhalb der transparenten Linse (18) erstreckt,
- ein aufblasbares Gurtzeug (20), das dazu ausgelegt ist, sich der Schale entgegengesetzt um den Kopf des Benutzers herum zu erstrecken, um die Schale am Kopf des Benutzers zu halten, wobei das Gurtzeug (20) zumindest einen hinteren Abschnitt (25) umfasst, der dazu bestimmt ist, einem Hinterkopfbereich des Kopfes des Benutzers gegenüberzuliegen,
- ein Führungsband (110), das ein oberes Ende (110a) und ein unteres Ende (110b) aufweist, wobei das obere Ende (110a) mit dem oberen Randabschnitt (19a) verbunden ist, und
- eine Platte (120), die mit dem unteren Ende (110b) des Führungsbandes (110) verbunden ist und eine Auflagefläche (120a) aufweist, die dazu ausgelegt ist, dem Hohlraum (13, 15) gegenüberliegend an der Schale (14, 17) anzuliegen, wobei die Platte (120) mit dem hinteren Abschnitt (25) des Gurtzeugs (20) verbunden ist,
- wobei das Führungsband (110) einen oberen Abschnitt (112) und einen unteren Abschnitt (114) aufweist, wobei der obere Abschnitt (112) starr ausgeführt ist und das Führungsband (110) dazu ausgelegt ist, mit dem oberen Abschnitt (112) auf die Platte (120) und mit dem unteren Abschnitt (114) zwischen dem oberen Abschnitt (112) und der Platte (120) umgelegt zu werden,
wobei das Verfahren **dadurch gekennzeichnet ist, dass** es umfasst:
a) Heranführen der Platte (120) an die Schale (14, 17),
b) Verschwenken des Führungsbands (110) in der Nähe seines oberen Endes (110a) in Bezug auf die Atemmaske (10),
c) Platzieren der Atemausrüstung in einer Verstauposition, indem das Führungsband (110) so umgelegt wird, dass der obere Abschnitt (112) des Führungsbandes (110), der untere Abschnitt (114) des Führungsbandes (110) und die Platte (120) aufeinanderliegen, und
d) Einführen der Atemausrüstung in die Aufbewahrungsbox.

## Claims

1. A breathing apparatus (100) for aircraft, the breathing apparatus being intended to supply a respiratory gas to a user and including:
- a breathing mask (10) comprising a shell (14, 17), the shell (14, 17) has a cavity (13, 15) and is configured for contacting the user's head around the user's mouth and nose, the shell (14, 17) supporting a transparent lens (18) and comprising an upper edge portion (19a) extending above the transparent lens (18),
- an inflatable harness (20) configured for extending around the user's head opposite the shell in order to hold the shell in contact with the user's face, the harness (20) comprising at least one rear portion (25) intended to be placed facing an occipital portion of the user's head,
- a guiding link (110) having an upper end (110a) and a lower end (110b), the upper end (110a) being linked to the upper edge portion (19a), and
- a plate (120) linked to the lower end (110b) of the guiding link (110) and having a bearing face (120a) configured for contacting the shell (14, 17) and facing the cavity (13, 15), the plate (120) being linked to the rear portion (25) of the harness (20),
wherein:
- the guiding link (110) has an upper portion (112) and a lower portion (114), the upper portion (112) being rigid,
the apparatus being **characterized in that**:
- the guiding link (110) is configured for being folded with the upper portion (112) on the plate (120) and the lower portion (114) between the upper portion (112) and the plate (120),
- the upper portion (112) has a length (L) greater than or equal to 10 centimetres,
- the upper portion (112) is rotatably mounted on the upper edge portion (19a), and
- the lower portion (114) is at least partly flexible and arranged between the upper portion (112) and the plate (120).

2. The breathing apparatus according to claim 1, wherein the guiding link (110) has a torsional flexibility of less than half a turn.

3. The breathing apparatus according to either one of the preceding claims, wherein the guiding link (110) has an elongate, flat shape.

4. The breathing apparatus according to any one of the preceding claims, wherein the upper portion (112) comprises two arms (112a, 112b) extending longitudinally and laterally separated from one another by a distance (d) greater than or equal to 3 centimetres and the breathing apparatus comprises two lower portions (114a, 114b) extending between the upper portion (112) and the plate (120).

5. The apparatus according to the preceding claim, wherein the lower portion (114) comprises at least one rigid element (115).

6. The breathing apparatus according to the preceding claim, wherein the lower portion comprises a series of rigid elements (115) hingedly connected to one another.

7. The breathing apparatus according to any one of the preceding claims, wherein the lower portion (114) comprises a strap, the strap extending beyond the lower end (110b) forming a loop (116) around a tube (21, 22) of the harness (20) and the loop (116) has ends (116a, 116b) held on the plate (120).

8. The breathing apparatus according to any one of claims 1 to 6, wherein the lower end (110b) of the guiding link (110) is linked to the rear portion (25) of the harness (20) via the plate (120).

9. The breathing apparatus according to any one of the preceding claims, wherein the breathing apparatus (110) is additionally provided with a sign (101, 102, 104, 105, 106, 107) for correct positioning in a stowage position.

10. The breathing apparatus according to any one of the preceding claims, wherein the shell (14, 17) comprises an oronasal face cover (14) and a support (17), the oronasal face cover (14) having a first cavity (13) and being configured for contacting the user's head around the user's mouth and nose, the support (17) supporting a transparent lens (18) and having a second cavity (15).

11. A breathing unit (1) comprising a breathing apparatus according to any one of the preceding claims and a stowage box (30) including a housing (32) configured for receiving the breathing apparatus (100) in a stowage position, said stowage box (30) having an opening (34) for access to the housing (32).

12. A method for stowing a breathing apparatus (1) for aircraft in a stowage box (30), wherein:
the breathing apparatus (100) and the stowage box (30) are comprised in a breathing unit (1), the stowage box (30) includes a housing (32) configured for receiving the breathing apparatus (100) in a stowage position, said stowage box (30) having an opening (34) for access to the housing (32),
the breathing apparatus is intended to supply a respiratory gas to a user and includes:
- a breathing mask (10) comprising a shell (14, 17), the shell (14, 17) has a cavity (13, 15) and is configured for contacting the user's head around the user's mouth and nose, the shell (14, 17) supporting a transparent lens (18) and comprising an upper edge portion (19a) extending above the transparent lens (18),
- an inflatable harness (20) configured for extending around the user's head opposite the shell in order to hold the shell in contact with the user's face, the harness (20) comprising at least one rear portion (25) intended to be placed facing an occipital portion of the user's head,
- a guiding link (110) having an upper end (110a) and a lower end (110b), the upper end (110a) being linked to the upper edge portion (19a), and
- a plate (120) linked to the lower end (110b) of the guiding link (110) and having a bearing face (120a) configured for contacting the shell (14, 17) and facing the cavity (13, 15), the plate (120) being linked to the rear portion (25) of the harness (20),
wherein:
- the guiding link has an upper portion (112) and a lower portion (114), the upper portion (112) being rigid and the guiding link (110) being configured for being folded with the upper portion (112) on the plate (120) and the lower portion (114) between the upper portion (112) and the plate (120),
the method being **characterized in that** it comprises:
a) moving the plate (120) to be in contact with the shell (14, 17),
b) pivoting the guiding link (110) near its upper end (110a) with respect to the breathing mask (10),
c) placing the breathing apparatus in a stowed position by folding the guiding link (110) so that the upper portion (112) of the guiding link (110), the lower portion (114) of the guiding link (110) and the plate (120) overlap, and
d) inserting the breathing apparatus into the stowage box.
